Europäisches Patentamt

European Patent Office

Office européen des brevets

(11) Veröffentlichungsnummer: **0 485 877 A1**

# EUROPÄISCHE PATENTANMELDUNG

(21) Anmeldenummer: **91118888.6**

(51) Int. Cl.5: **A61G 7/05, A61M 5/14**

(22) Anmeldetag: **06.11.91**

(30) Priorität: **10.11.90 DE 9015435 U**

(43) Veröffentlichungstag der Anmeldung:
**20.05.92 Patentblatt 92/21**

(84) Benannte Vertragsstaaten:
**AT BE CH DE DK ES FR GB GR IT LI LU NL SE**

(71) Anmelder: **B. Braun Melsungen AG**
**Carl-Braun Strasse**
**W-3508 Melsungen(DE)**

(72) Erfinder: **Reuter, Jürgen**
**Zum Espe 1**
**W-6445 Alheim-Heinebach(DE)**
Erfinder: **Heckmann, Harald**
**Unterster Weg 3**
**W-3503 Lohfelden 2(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) **Hängevorrichtung zur Befestigung eines Körperfluid-Sammelbehälters an einem waagerechten Holm.**

(57) Eine zuverlässig gesicherte Hängevorrichtung zur Befestigung eines Körperfluid-Sammelbehälters an einem waagerechten Holm besteht aus einem mit dem Körperfluid-Sammelbehälter verbundenen Bügelgriff (50), an dem zwei um den Holm herumlegbare Stränge einer Kordel (40) befestigt sind, wobei eine auf den abgeflachten Bügelgriff (50) passend aufsteckbare U-förmige Klammer (10) vorgesehen ist, an deren einem Schenkel (11) zwei zum Schenkelverlauf parallele Kanäle (24) zum Durchlaß je eines Kordelstranges (40a, 40b) ausgebildet sind, die an ihrem einen Endbereich jeweils einen Klemmspalt (30) für den Kordelstrang (40a,40b) aufweisen.

FIG.2

EP 0 485 877 A1

Die Erfindung bezieht sich auf eine Hängevorrichtung zur Befestigung eines Körperfluid-Sammelbehälters an einem waagerechten Holm, bestehend aus einem mit dem Körperfluid-Sammelbehälter verbundenen Bügelgriff, an dem zwei um den Holm herumlegbare Stränge einer Kordel befestigt sind.

Körperfluid-Sammelbehälter dienen in der Medizintechnik zur Aufnahme von Sekret oder Urin, das bzw. der über einen Schlauch von dem Patienten zu dem Sammelbehälter geleitet wird. Dabei muß für freihängende Befestigung des Körperfluid-Sammelbehälters, der nachfolgend "Behälter" genannt wird, gesorgt werden, und es ist eine für das Personal praktikable Art der Anbringung vorzusehen. Bisher wird die schlaufenförmige Kordel, deren Enden an dem Bügelgriff fixiert sind und deren geschlossene Schlaufe frei ist, nach Herumlegung ihrer beiden Stränge um den waagerechten Holm, z.B. eines Bettes, an diesem festgebunden, was umständlich und unsicher ist. Mit zunehmender Füllung des Behälters wird er schwerer und die auf der Kordel lastende Zugkraft kann ihre Verknotung lockern oder sogar lösen.

Bei einer Hängevorrichtung gemäß US-A- 30 90 968 werden die beiden geschlossenen Enden einer um einen Holm und um eine Griffstange des Behälters herumgelegten Kordelschlaufen mittels eines Hakenkörpers miteinander verbunden. Beim Anstoßen gegen den Behälter kann sich der Haken aushängen und der Behälter fällt von dem Holm ab.

Der Erfindung liegt die Aufgabe zugrunde, eine Hängevorrichtung der eingangs erwähnten Art so zu verbessern, daß die Kordel sich auf einfache Weise in ihrer Umschlingungsposition an dem Holm sichern läßt.

Diese Aufgabe wird erfindungsgemäß gelöst durch eine auf den abgeflachten Bügelgriff passend aufsteckbare U-förmige Klammer, an deren einem Schenkel zwei zum Schenkelverlauf parallele Kanäle zum Durchlaß je eines Kordelstranges ausgebildet sind, die an ihrem einen Endbereich jeweils einen Klemmspalt für den Kordelstrang aufweisen.

Die U-förmige Klammer, die vor ihrer Festlegung an dem Bügelgriff auf den beiden Kordelsträngen verschiebbar ist, bildet einen Klipp, dessen Klemmspalte nach Aufstecken des Klipps auf den Bügelgriff die Kordelstränge quetschen und damit gegen ein Durchrutschen sichern. Die schlaufenförmige Kordel wird nicht je nach Belieben vom Pflegepersonal um einen waagerechten Holm herumgeschlungen und festgeknotet, sondern sie wird gezielt einmal um den Holm herumgelegt und durch Aufstecken der Klammer auf den Bügelgriff festgelegt.

Dies ist einfach und geht schnell. Die Klemmspalte garantieren eine Sicherung der Kordel in festgezogener Position. Da der Bügelgriff abgeflacht ist, vorzugsweise ein durchgehend rechteckiges Profil hat, kann sich die U-förmige Klammer nicht auf dem Bügelgriff verdrehen.

Bei einem Ausführungsbeispiel der Erfindung ist vorgesehen, daß jeder Kanal in einer Röhre an je einem Rand des als Platte gestalteten einen Klammerschenkels ausgebildet ist, die in ihrem an den Bügelgriff anliegenden Wandungsbereich eine an einem Ende offene Längsaussparung von dem Kordeldurchmesser etwa entsprechender Breite aufweist und daß gegen das offene Ende der Längsaussparung ein Wandabschnitt des dieser gegenüberliegenden Wandungsbereiches der Röhre herabgezogen ist, dessen Rand mit der Oberfläche des Bügelgriffes einen Querschlitz bildet, dessen Höhe geringer als der Durchmesser der Kordel ist. In diesem Falle wird der Klemmspalt durch das Zusammenwirken der Oberfläche des Bügelgriffes mit dem Rand des herabgezogenen einen Wandungsbereiches der Röhre gebildet. Die Einquetschung jedes Kordelstranges in einem Querschlitz läßt sich noch dadurch vergrößern, daß die Kante des Randes des Wandabschnittes scharf und/oder gezackt profiliert ist.

Die Ausbildung des die Röhren tragenden Klammerschenkels als Platte vermittelt der Klammer gute Steifigkeit, so daß sich die Röhren unter dem Einfluß der von dem sich füllenden Behälter auf die Kordel ausgeübten Kraft nicht deformieren und die Klemmfunktion der Querschlitze gewährleistet ist. Bei dieser Ausbildung der erfindungsgemäßen Hängevorrichtung ist es günstig, daß die Klemmspalte sich an den dem geschlossenen Bogenteil der U-förmigen Klammer benachbarten Enden der Röhren befinden. Dabei soll günstigerweise nach Umschlingung des Holmes mit der Kordel der Bogenteil der auf den Bügelgriff aufgesteckten Klammer von dem Holm wegweisen und der als Platte gestaltete Klammerschenkel gegen die Oberseite des Bügelgriffes angesetzt sein. Die Kordel wird also nach 360°-Umschlingung des Holmes über den Bügelgriff hinweggezogen und auf der Oberseite des Bügelgriffes mit Hilfe der Klammer fixiert. Das zunehmende Gewicht des Behälters übt auf die Kordel Kraftkomponenten aus, die bei dieser Art der Klammerbefestigung den Anpreßdruck der Kante des den Querschlitz an einer Seite begrenzenden Wandungsbereiches jeder Röhre gegen die Kordel steigern.

Bei einer anderen vorteilhaften Ausgestaltung der Erfindung ist jeder Kanal in einer Röhre an je einem Rand des als Platte gestalteten einen Klammerschenkels ausgebildet, die in ihrem dem Bügelgriff abgewandten Wandungsbereich einen endseitig offenen und innen geschlossenen Längsschlitz aufweist, dessen Breite geringer als der Durchmesser der Kordel ist.

Nach Herumlegung der Kordel um einen waagerechten Holm werden die Kordelstränge in den jeweiligen Längsschlitz ihrer Röhre pressend hineingezogen und damit an der Klammer festgeklemmt, so daß die Kordel gegen Durchrutschen gesichert ist. Unter der Belastung des sich füllenden Behälters zieht sich die Kordel von selbst immer weiter in die beiden Längsschlitze hinein, so daß sich die Haltekräfte noch erhöhen. Diese Hängevorrichtung mit Klammer läßt sich einfach handhaben und gewährleistet durch zuverlässige Klemmsicherung der Kordel eine stabile Befestigung eines Behälters an einem waagerechten Holm. Da bei dieser Ausführungsform die in der Röhrenwandung ausgebildeten Klemmspalte von dem Bügelgriff unabhängig sind, können die Röhren den Bügelgriff in Querrichtung überragen, so daß die Längsschlitze eine für die zunehmende Einklemmung der Kordelstränge günstige Länge haben. Vorteilhafterweise werden die Längsschlitze gegen ihr inneres geschlossenes Ende schmaler, und die Keilwirkung verstärkt den Halteeffekt der Einklemmung. Die Einführungsöffnung der Längsschlitze ist vorzugsweise an den dem geschlossenen Bogenteil der U-förmigen Klammer benachbarten Enden der Röhren vorgesehen.

Nach Umschlingung des Holmes mit der Kordel weist der Bogenteil der auf den Bügelgriff aufgesteckten Klammer von dem Holm weg und der als Platte gestaltete Klammerschenkel ist gegen die flächige Unterseite des Bügelgriffes angesetzt. Anders als bei dem ersten Ausführungsbeispiel wird hierbei die den Holm um 360° umschlingende Kordel durch die Grifföffnung des Bügelgriffes gezogen, und es wird die Klammer von unten gegen den Bügelgriff angesetzt. Die Röhren befinden sich dann an der Unterseite des Bügelgriffes, und die Kordelstränge werden durch abwärts gerichtete Zugausübung in die Längsschlitze hineingezogen bzw. gedrückt. Diese Art der Kordelverlegung und Klammeranbringung ist handhabungstechnisch günstig, weil die Kordel nicht zwischen Holm und Bügelgriffoberseite hindurchgefädelt werden muß, und sie erzeugt für das Hineinziehen der Kordelstränge in die Längsschlitze günstige Kraftkomponenten.

In der Zeichnung sind zwei Ausführungsbeispiele der Erfindung schematisch dargestellt. Es zeigt:

Fig. 1 eine Seitenansicht der ersten Ausführungsform einer Klammer einer Hängevorrichtung,

Fig. 2 eine Frontansicht der Klammer nach Fig. 1,

Fig. 3 eine Draufsicht auf eine Hälfte der Klammer nach Fig. 1 und 2,

Fig. 4 die Hängevorrichtung mit der Klammer nach Fig. 1 bis 3 in montiertem Zustand,

Fig. 5 eine teilweise geschnittene Seitenansicht einer zweiten Ausführungsform einer Klammer einer Hängevorrichtung,

Fig. 6 eine Frontansicht der Klammer nach Fig. 5,

Fig. 7 eine Draufsicht auf eine Hälfte der Klammer nach Fig. 5 und 6 und

Fig. 8 die Hängevorrichtung mit der Klammer nach Fig. 5 bis 7 in montiertem Zustand.

Eine einstückige U-förmige Klammer 10 aus steifem Kunststoffmaterial besteht im wesentlichen aus einem als Platte gestalteten Klammerschenkel 11, an dessen eine Seite über einen geschlossenen gewölbten Bogenteil 12 ein Haken 13 angeformt ist, dessen Schenkel 14 zu dem Schenkel 11 im wesentlichen parallel verläuft und etwa gleiche Länge hat wie dieser. Die Klammer 10 ist durch zwei unterbrechungslose Wülste 15 auf der Außenfläche verstärkt. Die Platte des Klammerschenkels 11 ist etwa quadratisch und von ihrem einen Ende ausgehend verjüngt sich der Bogenteil 12 gegen den Schenkel 14, der parallelrandig ist. Der Klammerschenkel 11 wird an beiden Seitenrändern von zwei Röhren 20, 21 flankiert, die zueinander parallel verlaufen und viereckigen inneren und äußeren Querschnitt haben. Die beiden Röhren 20, 21 sind identisch. Die beiden Enden jeder Röhre 20, 21 schließen im wesentlichen bündig mit dem Rand des plattenartigen Klammerschenkels 11 bzw. der Ebene des geschlossenen Bogenteiles 12 ab.

Die Innenfläche der gesamten Klammer 10 ist glattflächig.

Jede Röhre 20, 21 enthält einen von dem einen offenen Ende 22 zum anderen offenen Ende 23 durchgehenden Kanal 24, der in der der Öffnung 22 zugehörigen Hälfte, die dem Bogenteil 12 abgewandt ist, etwa quadratischen gleichmäßigen Querschnitt hat und von vier zueinander senkrechten, ebenen Wandungsbereichen 25, 26, 27, 28 umgeben ist. Auf der anderen Hälfte der Röhren 20, 21 verjüngt sich der Kanal 24 gegen die Öffnung 23, weil der eine Wandungsbereich 25 über einen Wandabschnitt 25a gegen die Ebene des Wandungsbereiches 28 geneigt schräg verläuft. Die beiden Wandungsbereiche 26 und 27 sind entsprechend der Schräge des Wandbereichabschnittes 25a abgeschrägt. Die axiale Länge des Wandungsbereiches 28 ist kürzer als die Hälfte der Röhrenlänge. An seinen inneren geraden Rand 28a schließt sich eine Längsaussparung 29 an, deren Breite dem Abstand der Innenflächen der beiden Wandungsabschnitte 26 und 27 entspricht.

In dem schrägen Wandabschnitt 25a des Wandungsbereiches 25 ist ein gegen die Kanalöffnung 23 bei 30b offener, gerader Längsschlitz 30 ausgebildet, der am inneren Ende des schrägen Wandbereichabschnittes 25a bei 30a etwa auf der Quermitte der Röhre 20, 21 geschlossen ist. Der Längsschlitz 30 verjüngt sich etwas gegen das geschlossene Ende 30a. Er befindet sich in der Längsmitte des Wandungsbereiches 25.

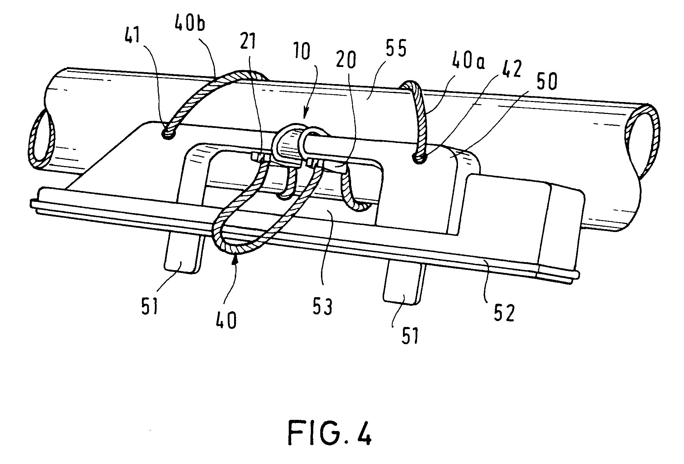

Die Klammer 10 dient zur Fixierung einer Kordel 40 aus geflochtenen oder in anderer Weise verschlungenen Fäden an einem Bügelgriff 50 mit rechteckigem Querschnitt, wobei die größere Achse des Rechteckes in der Waagerechten verläuft. Die beiden freien Enden 41, 42 (Fig. 4) der zu einer Schlaufe gelegten Kordel 40 sind z.B. durch Verknotung an dem Bügelgriff 50 befestigt. Der Bügelgriff 50 ist mit Tragelementen 51 an einem Balkenteil 52 zur Anbringung eines der Deutlichkeit halber nicht gezeichneten Sammelbeutels für Urin oder dergleichen Körperfluid ausgerüstet. Die Klammer 10 ist durch Hindurchziehen der beiden Stränge 40a und 40b der Kordel 40 durch je eine Röhre 20, 21 der Klammer 10 auf der Kordel 40 verschiebbar aufgefädelt. Die Kordel 40 wird um einen waagerechten Holm 55 mit z.B. kreisförmigem Querschnitt so herumgelegt, daß die beiden Kordelstränge 40a, 40b von oben her um den Holm 55 herumgelegt und durch das Griffloch 53 des Bügelgriffes 50 hindurchgezogen sind. Die Klammer 10 wird dann so gegen den Bügelgriff 50 angesetzt, daß der plattenförmige Klammerschenkel 11 mit den beiden Röhren 20, 21 sich auf der Unterseite des Bügelgriffes 50 befindet und der Schenkel 14 oben liegt. Der Bogenteil 12 ist von dem Holm 55 weggewandt und dem Hantierenden zugekehrt. Durch Festhalten der aufgesteckten Klammer 10 läßt sich durch Zug an der Schlaufe der Kordel 40 diese festspannen. Sodann werden die beiden Stränge 40a, 40b durch Nachunterziehen in die Längsschlitze 30 hineingepreßt, wodurch sich eine Arretierung und Durchrutschsicherung für die Kordel 40 ergibt.

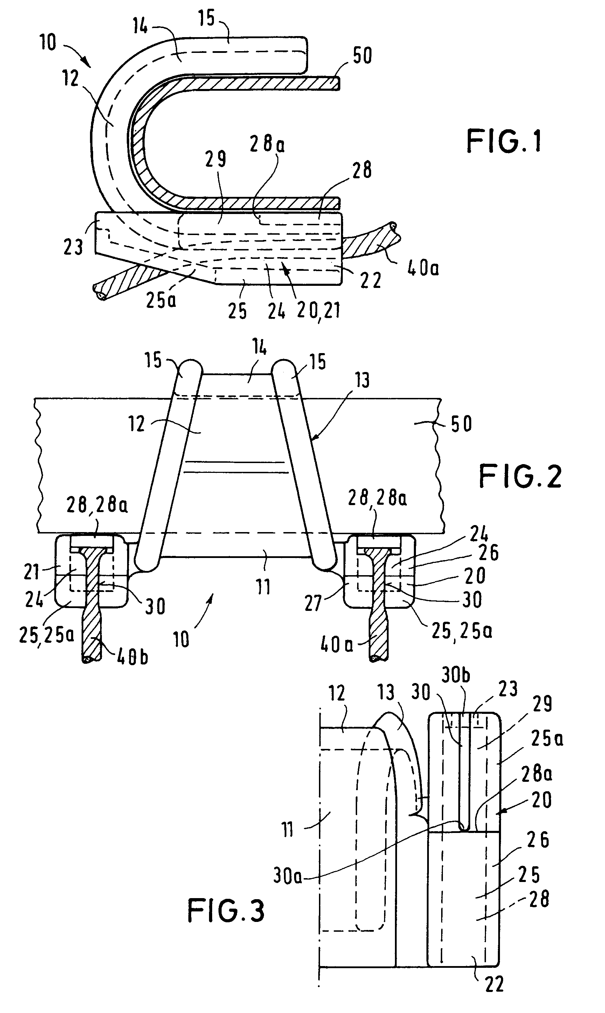

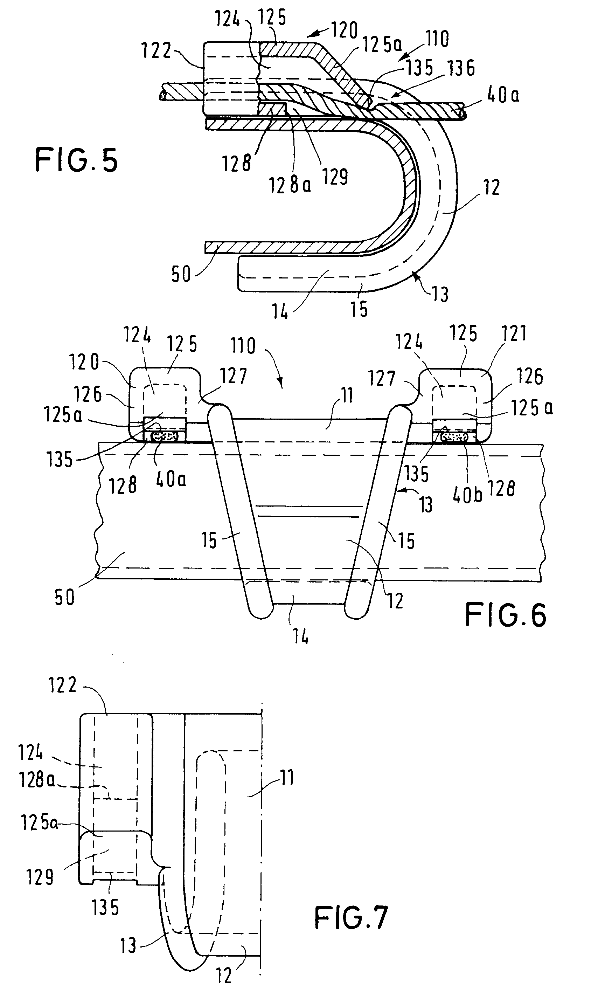

Bei dem Beispiel der Figuren 5 bis 8 sind gleiche Teile der zweiten Ausführungsform der Hängevorrichtung mit den gleichen Bezugszeichen wie bei dem Beispiel der Figuren 1 bis 4 bezeichnet. Bei abgewandelten Teilen sind die Bezugszeichen durch "100" ergänzt.

Während bei dem ersten Ausführungsbeispiel die Klemmspalte durch Längsschlitze 30 in dem Wandungsbereich 25 der Röhren 20, 21 gebildet sind, bestehen sie bei dem zweiten Beispiel aus je einem Teil der Röhren 120, 121 und der diesem gegenüberliegenden Fläche des Bügelgriffes 50.

Die einstückig aus Kunststoffmaterial geformte U-förmige Klammer 110 besteht ebenfalls aus einem als Platte gestalteten Klammerschenkel 11, an dessen eine Seite über einen geschlossenen gewölbten Bogenteil 12 ein Haken 13 angeformt ist, dessen äußerer Schenkel 14 zu dem Schenkel 11 im wesentlichen parallel verläuft und gleiche Länge hat wie dieser. Die Klammer 110 ist durch unterbrechungslose Wülste 15 auf der Außenfläche verstärkt. Die quadratische Platte des Klammerschenkels 11 wird an einem Ende von dem sich verjüngenden Bogenteil 12 fortgesetzt, der in den parallelrandigen Schenkel 14 übergeht. Der Klammerschenkel 11 ist an beiden Seitenrändern von je einer Röhre 120, 121 flankiert, die zueinander parallel verlaufen und je einen Kanal 124 enthalten. Jeder Kanal 124 hat über den größten Teil seiner Länge viereckigen, vorzugsweise quadratischen Querschnitt, und er ist von Wandungsbereichen 125, 126, 127 und 128 begrenzt. Die dem Bogenteil 12 des Hakens 13 abgewandte Öffnung 122 jedes Kanals 124 ist viereckig und verläuft quer zur Ebene der Schenkel 11 und 14. Die andere Öffnung besteht aus einer Längsaussparung 129 in dem Wandungsbereich 128, die von den Längsrändern der beiden Wandungsbereiche 126 und 127 sowie dem quergerichteten Rand 128a des verkürzten Wandungsbereiches 128 und dem quergerichteten Rand eines Wandabschnittes 125a begrenzt ist, der von dem Wandungsbereich 125 in Richtung des Wandungsbereiches 128 abgeknickt ist. Der Rand des Wandabschnittes 125a liegt mit einer scharfen Kante 135 etwa in der Ebene der Innenfläche des Wandungsbereiches 128.

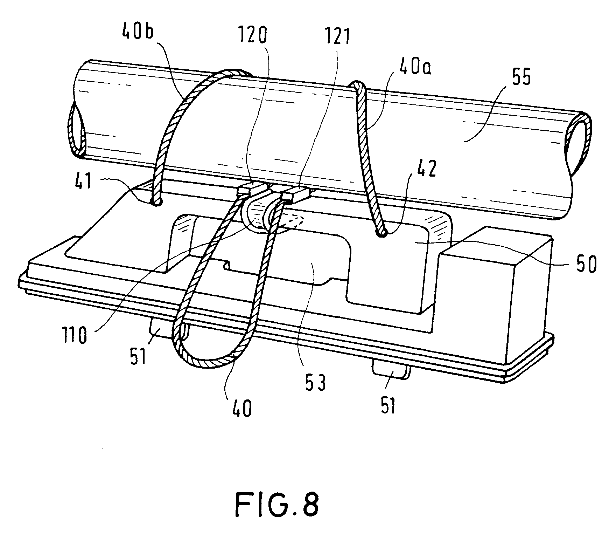

Die Klammer 110 steckt verschiebbar auf der Kordel 40. Die beiden Stränge 40a, 40b sind durch die Röhren 120, 121 hindurchgezogen. Sie treten durch die Öffnungen 122 in die Röhren 124 ein und durch die Längsaussparung 129 aus diesen wieder aus. Die Kordel 40 wird ausgehend von ihren Befestigungsstellen 41, 42 an dem Bügelgriff 50 über den Holm 55 gelegt und mit ihrem geschlossenen Ende zwischen dem Holm 55 und der Oberseite des Bügelgriffes 50 hindurchgezogen (Fig. 8). Die Klammer 110 wird in Richtung des Holmes 55 auf den flachen Bügelgriff 50 aufgesteckt. Dabei werden die beiden Kordelstränge 40a, 40b zwischen der scharfen Kante 135 und der Oberfläche des Bügelgriffes 50, gegen die der Wandungsbereich 128 jeder Röhre 120, 121 anliegt, fest eingequetscht, d.h. die Kante 135 bildet mit der Oberfläche des Bügelgriffes 50 einen Klemmschlitz 136, der ein Durchrutschen der Kordelstränge 40a, 40b verhindert.

**Patentansprüche**

1. Hängevorrichtung zur Befestigung eines Körperfluid-Sammelbehälters an einem waagerechten Holm, bestehend aus einem mit dem Körperfluid-Sammelbehälter verbundenen Bügelgriff, an dem zwei um den Holm herumlegbare Stränge einer Kordel befestigt sind,
**gekennzeichnet durch**
eine auf den abgeflachten Bügelgriff (50) passend aufsteckbare U-förmige Klammer (10;110), an deren einem Schenkel (11) zwei zum Schenkelverlauf parallele Kanäle (24;124) zum Durchlaß je eines Kordelstranges (40a, 40b) ausgebildet sind, die an ihrem einen Endbereich jeweils einen Klemmspalt (30;136) für den Kordelstrang (40a, 40b) aufweisen.

2. Hängevorrichtung nach Anspruch 1,
**dadurch gekennzeichnet,** daß
jeder Kanal (124) in einer Röhre (120, 121) an je einem Rand des als Platte gestalteten einen Klammerschenkels (11) ausgebildet ist, die in ihrem an den Bügelgriff (50) anliegenden Wandungsbereich (128) eine an einem Ende offene Längsaussparung (129) von dem Kordeldurchmesser etwa entsprechender Breite aufweist und daß gegen das offene Ende der Längsaussparung (129) ein Wandabschnitt (125a) des dieser gegenüberliegenden Wandungsbereiches (125) der Röhre (120,121) herabgezogen ist, dessen Rand mit der Oberfläche des Bügelgriffes (50) einen Querschlitz (136) bildet, dessen Höhe geringer als der Durchmesser der Kordel (40) ist.

3. Hängevorrichtung nach Anspruch 2,
**dadurch gekennzeichnet,** daß
die Kante (135) des Randes des Wandabschnittes (125a) scharf und/oder gezackt profiliert ist.

4. Hängevorrichtung nach Anspruch 1,
**dadurch gekennzeichnet,** daß
jeder Kanal (24) in einer Röhre (20,21) an je einem Rand des als Platte gestalteten einen Klammerschenkels (11) ausgebildet ist, die in ihrem dem Bügelgriff (50) abgewandten Wandungsbereich (25) einen endseitig offenen (30b) und innen geschlossenen (30a) Längsschlitz (30) aufweist, dessen Breite geringer als der Durchmesser der Kordel (40) ist.

5. Hängevorrichtung nach einem der Ansprüche 1 bis 4,
**dadurch gekennzeichnet,** daß
die Klemmspalte (30;136) sich an den dem Bogenteil (12) der U-förmigen Klammer (10;110) benachbarten Enden der Röhren (20,21; 120,121) befinden.

6. Hängevorrichtung nach einem der Ansprüche 1 bis 5,
**dadurch gekennzeichnet,** daß

nach Umschlingung des Holmes (55) mit der Kordel (40) der Bogenteil (12) der auf den Bügelgriff (50) aufgesteckten Klammer (110) von dem Holm (55) wegweist und der als Platte gestaltete Klammerschenkel (11) gegen die Oberseite des Bügelgriffes (50) angesetzt ist.

7. Hängevorrichtung nach einem der Ansprüche 1 bis 5,
**dadurch gekennzeichnet,** daß
nach Umschlingung des Holmes (55) mit der Kordel (40) der Bogenteil (12) der auf den Bügelgriff (50) aufgesteckten Klammer (10) von dem Holm (55) wegweist und der als Platte gestaltete Klammerschenkel (11) gegen die Unterseite des Bügelgriffes (50) angesetzt ist.

FIG.1

FIG.2

FIG.3

FIG. 4

FIG.5

FIG.6

FIG.7

FIG.8

**Europäisches Patentamt**

# EUROPÄISCHER RECHERCHENBERICHT

Nummer der Anmeldung

EP    91 11 8888

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A, D | US-A-3 090 968 (BUONO) <br> * das ganze Dokument * <br> --- | 1 | A61G7/05 <br> A61M5/14 |
| A | US-A-3 568 965 (CLARK) <br> * das ganze Dokument * <br> --- | 1 | |
| A | US-A-4 332 252 (TAYLOR) <br> * das ganze Dokument * <br> ----- | 1 | |
| | | | **RECHERCHIERTE SACHGEBIETE (Int. Cl.5)** <br><br> A61G <br> A61M <br> A61F <br> F16B <br> F16G <br> F16M |

Der vorliegende Recherchenbericht wurde für alle Patentansprüche erstellt

| Recherchenort | Abschlußdatum der Recherche | Prüfer |
|---|---|---|
| DEN HAAG | 04 FEBRUAR 1992 | BAERT F. |